# EUROPEAN PATENT SPECIFICATION

(11) **EP 3 492 138 B1**
(45) Date of publication and mention of the grant of the patent: **23.08.2023**
(21) Application number: 18209386.4
(22) Date of filing: 30.11.2018
(51) Int. Cl.: A61N 1/36, A61H 19/00

(54) **ELECTRICAL STIMULATOR APPARATUS WITH CONTACTLESS FEEDBACK FROM A USER**
ELEKTRISCHE STIMULATORVORRICHTUNG MIT KONTAKTLOSER RÜCKMELDUNG VON EINEM BENUTZER
APPAREIL STIMULATEUR ÉLECTRIQUE DOTÉ D'UN RETOUR D'INFORMATIONS SANS CONTACT D'UN UTILISATEUR

(30) Priority: 30.11.2017 GB 201719988; 04.10.2018 GB 201816187
(43) Date of publication of application: 05.06.2019
(73) Proprietor: Cyrex Limited, Hoddesdon, Hertfordshire EN11 0FF (GB)
(72) Inventor: SMITH, Andrew James, Hoddesdon, Hertfordshire EN11 0FF (GB)
(74) Representative: Pure Ideas Limited

(56) References cited:
- EP-A1- 2 529 786
- EP-A1- 2 901 999
- EP-A1- 3 311 879
- GB-A- 2 499 585
- US-A1- 2013 023 954
- US-A1- 2015 022 328
- US-A1- 2016 216 768
- H HIGA ET AL: "A Video-Based Control Command Input Device for FES System", BIOMEDICAL CIRCUITS AND SYSTEMS CONFERENCE, 2007. BIOCAS 2007. IEEE, 1 November 2007 (2007-11-01), pages 103-106, XP055573527, Piscataway, NJ, USA DOI: 10.1109/BIOCAS.2007.4463319 ISBN: 978-1-4244-1524-3

## Description

### Field of the invention

The present invention relates to stimulator apparatus for applying stimulation to a user. The invention is particularly applicable, but in no way limited, to apparatus for stimulating penile, scrotal, anal, vaginal and clitoral tissue either by electrically or physical contact.

### Background to the invention

It is well known that the application of stimulation, electrical or physical such as vibration, thrusting, stroking or patting, to certain neuromuscular areas in or near the genitalia can be used to cause sexual arousal and can induce orgasm. There are a wide range of devices and apparatus available which can be used to apply the necessary stimulation to the subject areas. Such apparatus inevitably includes electrodes with electrical stimulation which are spaced apart by means of some type of insulator and which are placed in contact with the living tissue which is to be stimulated. Typically, existing electrodes are in the form of rings capable of transmitting low levels of electricity to the skin, nerves and muscle and which are applied to the penis or scrotum. Alternatively, a probe type of electrode is used to stimulate the skin inside and surrounding the vagina or anus.

With physical stimulation generally so called sex toys are used to provide the stimulation by contact consistently, periodically and/or randomly as required.

These devices require a controller, or an electrical stimulator to provide an electrical signal between an anode and a cathode for electrical stimulation or control signals for a physical control of the frequency, amplitude/severity and pulse duration.

An example of an electrical stimulator is the Power Box sold by Paradise Electro Stimulations Inc of 1509 West Oakley Blvd, Las Vegas, NV 89102, USA. Such electrical stimulators currently have limited functionality. They would typically have an on/off switch and some means for the operator to manually control the stimulation parameters applied to the electrodes. Such parameters include current, voltage, pulse frequency, and pulse width. This manual control is typically by means of one or more rotatable dials/potentiometers. The operator can adjust these settings during use, but this must be done manually, as and when required. Pre-programmed stimulators also exist, which automatically change the stimulation parameters in order to produce pulsing and waving patterns of electrical stimulation. The Erostek model ET-312 sold by ECForbes Inc of 4320 Redwood Highway, Suite 400, San Rafael CA 94903 USA, and ElectraStim models EM80/EM140 sold by Cyrex Ltd of 36 Hodesdon Industrial Centre, Pindar Rd, Hoddesdon EN11 0FF UK are programme controlled electrical stimulators. More recently such functionality has been further enhanced by Cyrex as outlined in GB2499585 by use of a movement sensor adapted to detect movement or physical motion of the housing and with control to varying amplitude and frequency from the movement sensor in direct sympathy with applied physical motions in order to vary one or more stimulating parameters of the electrical output of the stimulator apparatus. This control by some motion by an operator or the user of the sensor has greatly improved responsiveness to user's desires. However, by this prior approach it is still necessary to have some direct means of achieving direct motion of the housing which can be limiting in some situations.

There are numerous forms of sex toy some generally in the shape of a suitable phallic or phallus nature for physical contact and/or penetration as required. The necessary controls of wireless control can be so miniaturised that one way or two way control with the sex toy can be provided. An example of a sex toy is the Sybian device. However, in the course of intimacy there is a desire not to need to use an actual remote controller handset as this may interfere and interrupt the course of stimulation.

### Summary of the Invention

According to a first aspect of the present invention there is provided a stimulator apparatus according to claim 1.

Other features of aspects of the present invention are outlined in the dependent claims to claim 1 below.

### Brief Description of the Drawings

Preferred embodiments of the present invention will now be described, by way of example only, with reference to the accompanying drawings wherein:-
Figure 1 illustrates a control housing of an electrical stimulator apparatus;
Figure 2 illustrates a control housing relative to a user and an object or body part used for control in accordance with aspect of the present invention; and,
Figure 3 illustrates in a block diagram format one method by which a signal from a movement detector can be processed.

### Description of the preferred embodiments

The present invention will now be described by way of example only and with reference to the accompanying drawings. These are not the only ways that the invention may be put into practice, but they are the best ways currently known to the applicant.

Electrical stimulating machines are widely available in the medical field and are often called TENS (Transcutaneous Electronic Nerve Stimulator) and are used to apply a stimulating electrical current to the human body in order to reduce pain symptoms, or at higher intensity levels to force involuntary muscle contractions. These electrical stimulating machines provide a neuromuscular stimulation (NMS). The stimulating current generated by the NMS is applied to the human body by a wide variety of electrode designs which are either placed in contact with the surface of the skin or inserted into the genitals. It will be appreciated that physical stimulation whether it be vibration, stroking, penetration or patting effectively emulates the user's body response to electrical stimulation.. The physical stimulation can heighten the neuromuscular stimulation and vice versa.

In general, NMS devices, whether used for medical purposes or for recreational pleasure, do not have any form of input stimulus. The output or stimulating parameters on known NMS devices are controlled by switches and/or rotational control devices (potentiometers, digital encoders and the like) located on a control panel on the NMS. This is similar to physical simulators in that need to vary the period, intensity and frequency of physical simulation.

Key variable stimulating electrical parameters include the following:
- Current (typically 0 to 125 milliamps peak or more)
- Voltage (typically 0 to 60V peak into a 560R ohm load)
- Pulse Frequency (typically 20Hz to 150Hz)
- Pulse Width (typically 50 micro seconds to 200 micro seconds)

All of the above electrical parameters can be varied or modulated in order to generate pulsing or waving sensations at the site of stimulation. These pulsing and waving sensations are similar to those provided directly to a user by physical stimulation. These sensations are the result of the stimulations applied but each individual user is different and their respective desires will vary. In such circumstances means of control will ideally provide access for control and variation of the stimulation. Aspects of the present invention allow access to a range of controls such as proximity, gestures, colour and orientation which are associated with particular stimulations and/or stimulation regimes.

An object sensor with suitable detectors is provided by Avago Technologies under the product definition APDS-9960 and known as `Digital Proximity, Ambient Light RGB and Gesture Sensor'. Details can be found at https://www.broadcom.com/products/optical-sensors/integrated-ambient-light-and-proximity-sensors/apds-9960. This object sensor is mounted in a housing or enclosure with other associated electronic control circuitry including a micro-controller circuit unit (MCU) and a stimulation signal driver circuit. Connections are provided to the electronics and drivers for the electrodes to provide the stimulations as electrical stimulation parameters to the user. The object sensor can be oriented for context relative to a user in terms of a longitudinal and lateral orientations. Once the sensor has determined a control such as proximity, a gesture, movement or a colour of an object alone or in combination with other controls to vary or modulate a stimulation parameter or a succession of such stimulation parameters. A cabled connection is typically with aspects of the present invention. All of the electrodes are currently connected to the stimulator via a compatible cable. The cables plug into the stimulator at the top of a control housing.

As indicated multi-function gesture/proximity sensors are widely available, such as the APDS-9960 made by Broadcom/Avago Technologies. These devices advantageously include gesture detection, proximity detection, digital ambient light sensing (ALS) and colour sense (RGBC). As will be understood the sensor needs to be small and discreate - the APDS-9960 has a size about L 3.94 × W 2.36 × H 1.35 mm and also incorporates an IR LED and factory calibrated LED driver.

The use of apparatus in accordance with aspects of the present invention allows control but without physical contact between the control housing after it is switched on and of course at the end of activities switched off. In such circumstances the following forms of sensing can be utilised in accordance with aspects of the present invention either alone or in combinations.

### Proximity Sensing

The proximity function will be used to vary/modulate the nerve/muscle stimulation parameters, be that electrical and/or physical, in direct proportion to the proximity of an object or body part within the detection range of the sensor, typically in the order of 0 to 100mm in distance when in the direct field of sensing. With no object within the detection range there is no variance in the stimulation parameter. The modulated parameters could include one or more of the following stimulation parameters:-
1. Pulse duration - calibrated to be in the range of 50us to 200us
2. Pulse frequency - calibrated to be in the range of 10Hz to 200Hz
3. Pulse amplitude - calibrated to be in the range of 0 to 125mA

The object could be an item familiar to a user such as a toy or a household item and body parts may be fingers or hands. Steady presentation or flickering or movements of these objects or body parts will be 'seen' by the object sensor in order to initiate the modulated parameters in a sequence as determined previously and embedded within the apparatus and/or determined or adjusted for an individual user's requirements. It will be understood that the user may be aware of the object or body part by sight or presentation of the object or body part conducted blind to the recipient of the modulated parameters. It will also be understood that the body part or specific object could be an identifier for the recipient of the modulated parameters or another user of the apparatus or a type of user specified by or a need for a particular series of modulated parameters. In any event the determination of the proximity of the object or body part by the sensor is determined to give access to the modulated parameters.

In most circumstances the orientation or angle of presentation of the object or body part will not be too important provided the sensing parts of the apparatus can determine the object or body part.. The most important feature is that the object or body part is determined to activate the modulated parameter or sequence of parameters as required by a user. The sensor can monitor the approach and exit direction of motions by the use of multiple integrated IR sensors to determine each of the 6 pre-defined motions. The motions can be from a hand or other body part or a solid object like a book. Similarly, the enclosure in which the sensor is mounted could move past, near or far, a detected object to give rise to a detected gesture.

A possible feature of aspects of the present invention are that the actual modulated parameter or series of parameters can be learned for a particular user. For example, a user may gain particular pleasure with a modulated parameter or series of parameters and that modulated parameter or series of parameters referenced or indexed relative to an object or body determinable by the sensor of the apparatus. Furthermore, such particular pleasure may be associated with another user or a time or place or occasion so again referenced by an object or body part determinable by the apparatus. Finally, if the apparatus could be used to record the series of stimulations which occurred with another user by other means then these could be recorded and associated with that user and the apparatus could attempt to replicate such simulations when that other user was not available. The apparatus allows experimentation by a user as to the modulated parameter or series of parameters to find those which give particular pleasure to a user and then allow ready access to repeat such modulated parameter or series of parameters through a ready access metaphor in terms of an object or body part determinable by the sensor part of the apparatus.

### Gesture Sensing

The gesture function is used to detect 6 different types of motion within the sensing range as follows - left, right, up, down, towards, away. These motions are not detected in a linear fashion like proximity detection but simply as one of six recognised motions. Each detected motion can be used to vary stimulation parameters by increasing or decreasing stimulation parameters. The modified parameters in response to detected gestures would include the following as previously similarly to simple proximity as described above :-
1. Pulse duration -in the range of 50us to 200us
2. Pulse frequency - in the range of 10Hz to 200Hz
3. Pulse amplitude - in the range of 0 to 125mA
   but also can allow additionally these features:-
4. Move to next Function
5. Move to next Pattern
6. Switch Apparatus off

Thus, the object or body part proximity alone and/or a gesture could initiate pulsing and then a gesture (repeated or different) could provide for changes in function or pattern or switching off. For instance, a left direction hand motion in the detection range could increase the intensity of CH1 output by 1 or more levels, a right direction hand motion could increase the intensity of CH2 output by one or more levels. An up motion could increase both outputs and a down motion could decrease both outputs. A towards motion might change the Functions and an away motion might change a or the stimulation pattern. Gestures are a unique way of controlling the stimulation apparatus without physical contact once it has been switched on.

### Colour Sensing

The utilised sensor can also have the ability to detect levels of primary colours which can be used as another method of varying stimulation parameters or navigating the user interface without physical contact. For instance, the detection of the colour red might increase the intensity of CH1 output, the detection of Green might increase CH2 output and Blue might decrease them both. By analysing the RGB levels, typically by pre-determined software, it is also possible to detect other colours thus enabling multiple stimulation parameters to be controlled by the detection of colours as well as control functions as listed below...
1. Pulse duration -in the range of 50us to 200us
2. Pulse frequency - in the range of 10Hz to 200Hz
3. Pulse amplitude - in the range of 0 to 125mA
4. Move to next Function
5. Move to next Pattern
6. Switch Apparatus off

In use the stimulation apparatus would be placed in front of the coloured object and within the detection range of 100mm/4". The coloured object could simply be a piece of paper or card but to provide a more creative user experience a slide show of colours would provide a method of dynamically creating patterns on a computer screen, tablet or smart phone. It will also be understood that a program of different colours could be presented to the sensor on the screen of a tablet device.

The above features are unique techniques in stimulation and also have applications for use where limited dexterity exists.

As well as the interactive features described above, the apparatus will also have multiple selectable patterns programmed into memory, motion features using an accelerometer and audio input features whereby stimulation is varied/modulated by sounds within the audio spectrum. When not using contactless features as above the intensity can be adjusted by means of tactile switches, rotary encoders or potentiometers. The apparatus will incorporate a display for menu navigation and stimulation parameter display.

The present invention in preferred embodiments provides a control housing for an object sensor to detect at least one of proximity, gesture, movement and colour of an object as described above. Electrodes for electrical stimulation in accordance with a parameter or parameter in a sequence may be connected by wires with the control housing or using a wireless connection with possibly a separate housing and the control unit having different power supplies. Electrodes are generally physically connected via a cable to the control circuitry within a main control housing or enclosure/casing. Wireless control allows use of smart phones using Bluetooth connection protocols and means.

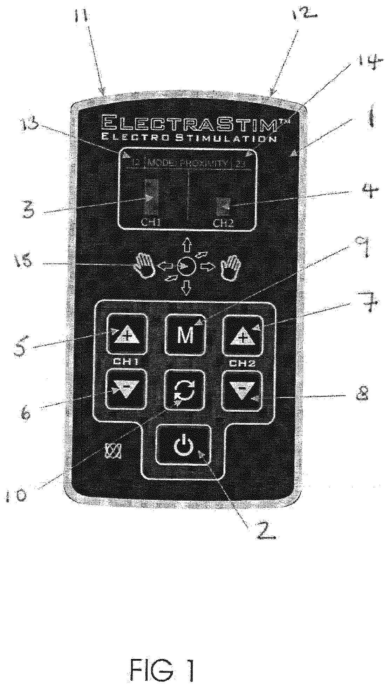

Figure 1 provides an illustration of a control housing 1 in accordance with aspects of the present invention. For convenience the housing 1 includes status features such as an on-off switch 2 and a graphic indicator 3 for channel CH1 along with a numeric display 13 and a graphic indicator 4 for channel CH2 along with a numeric display 14 . Pairs of switches 5, 6 and 7, 8 provide respective means to adjust intensity of stimulation up and down for channel CH1 and CH2.

A mode switch 9 is provided to change the control feature (proximity, gesture, movement and colour) and a program switch to allow select/choice of stimulation parameter or parameter program by a user. With the housing a symbolic display 15 with various arrowheads provides a graphic and symbolic display of movement of the object or body part as a hand but of course could be any object or body part used for control An object sensor or sensors are located to detect at least one control features of proximity, gesture, movement or colour of an object or body part. The sensors are positioned normally within the housing 1. The housing 1 may be arranged to have a known orientation to allow direction of operation of the apparatus but more importantly the sensor or sensors must be able to see an object visually or if also using radio frequency tagging or the like determine the object or body part. Normally only one sensor in accordance with aspects of the present invention will be used with possibly a viewing aperture which defines an arc within which the object or body part can be detected with respect to the control features of proximity, gesture, movement and/or colour. If only one object sensor is used then, as indicated above, the housing 1 may have a orientation marked in some way or inherently (the housing as a handset having the switches grouped appropriately so the user naturally 'points' the housing as required in one direction) so that a user can point the housing towards an object or where an object or body part is expected. Thus, different objects can be pre-arranged as required and the housing moved as necessary to 'view' each object as required. An alternative could be to provide an object sensor which can view a full 360 degree range about the housing. Thus, all objects or several object sensors view respective arcs or ranges about the housing so that collectively all or a desired viewing range is provided about the housing. For example, if the housing is placed to the side of a user then generally one side of the housing will be adjacent to a user whilst the other (open) side away from the user could define the arc within which an object or body part can be placed to provide the control feature (proximity, gesture, movement or colour).

An object or body part will be detected within a predetermined motion detection range so that proximity, gesture, movements and colour are determinable. These objects or body parts or portions thereof correspond to controls so that stimulation parameters alone or in sequence are provide through the electrodes and/or by direct physical contact as outlined above. It will be understood that the motion detection range is a function of the sensor. This range can be up to 10 centrimeters (100 milliimetres) but it can be greater with the problem then of inadvertent detection of object and/or body parts when these detections are not relevant for control or modulation of the stimulation parameters presented through the electrodes.

It will be understood if two or more object sensors are provided then each can be directed at a specific object or objects. For example, respective users may present different objects or body parts at each viewing aperture for each sensor so one user may present an object such as a book and another user present a body part with control circuitry within the control housing then arranged to provide the appropriate stimulation parameters to one or both users. Similarly, one sensor may simply determine proximity of any object including just that a user is present whilst the other sensor or sensors then detecting a specific object.

As indicated the object sensors in accordance with aspects of the present invention can determine at least one of proximity, gesture, colour or movement of an object or body part. Operation of the apparatus may require continued presence of the object or body part for the stimulation parameter through the electrodes. Thus, the object or body part is shown once then a stimulation parameter or sequence of parameters executed over a time period. If the object or body part continues to be seen by the sensor then the stimulation parameter or sequence through the electrodes continues but if removed it will stop or at least continue with the stimulation parameter for an agreed pre-determined time period after removal from detection of the object by the sensor. If the object is returned to be detected by the sensor then mere return may increase or decrease the intensity of the stimulation parameter or sequence or re-start the stimulation parameter agreed time period. If another object or body part is then presented to be seen and detected by the sensor then as indicated the stimulation parameter may be altered or there is a change in the stimulation parameter or sequence to the electrodes and/or direct physical contact.

Sensors in accordance with aspects of the present invention will typically have the capacity to detect more than one of the control features of proximity, gesture, colour and movement. The sensor and particularly the control circuity will then be able to determine distinct combinations of object, movements, gestures and colours to provide access to/execution of stimulation parameters and sequences of parameters to the electrodes or means of physical contact. Some control features such as simple up down movement may simply act to increase or left-right movements decrease stimulation parameters. The simple size of the object would provide a base level of stimulation parameter intensity so if the object or body part is brought closer or further way from the control housing this will increase or decrease intensity. Different objects, colours and gestures will simply allow easy access to respective stimulation parameters or sequences of stimulation parameters for the electrodes and/or means of physical contact.

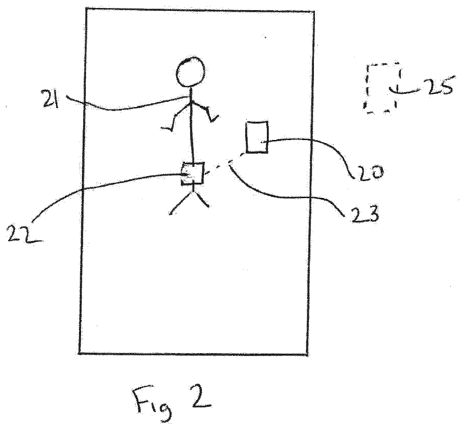

Figure 2 provides a simple schematic illustration of a control housing 20 in accordance with aspects of the present invention within a predetermined range of a user 21. The control housing 20 either is in wireless communication with a potential stimulation site 22 or through a wired connection (shown by broken line 23). Communication is normally one way with a master and slave approach - The controller (master) responding to the control feature or features as signals sent to the device for stimulation (electrical and/or physical).Once switched on or if on standby the control housing 20 is in a position to determine if an object or body part is presented or moved within a pre-determined range of the housing 20 where upon the potential stimulation site 22 will provide the stimulation parameters to a user through the electrodes and/or means of physical contact. The range may be up to 10cm provided there is not interference with inadvertent presence of objects or body parts within the range.

Normally there will only be one control or sensor housing 20 in accordance with aspects of the present invention. It will also be understood that more sensor or control housings (shown by broken lines 25) could be provided with greater or lesser ranges to a primary sensor housing 20. Such that one or both sensor housings through appropriate control circuity or objects/body parts or simply other environmental factors could provide or alter the stimulation parameters presented to the electrodes and/or means of physical contact. For example, the primary control housing 20 with its sensor or sensors is active but a secondary control housing 25 has other objects/body parts presented by another user to alter the stimulation parameter presented to a user 21 by the stimulation device and electrodes/means of physical contact. The secondary control housing could be wired or wirelessly as required and/or may even be accessed via a mobile device or computer remote from the location of the user. The secondary housing and its sensor 25 may simply have a modifier for operation of the primary controller so an ability to determine though colour a rough determination of night or day (dark or light) or identification of an object which acts as an on off switch. An object is detected by the secondary housing and stored whereby if that object is presented to the primary housing then stimulation to the electrodes is switched on or off.

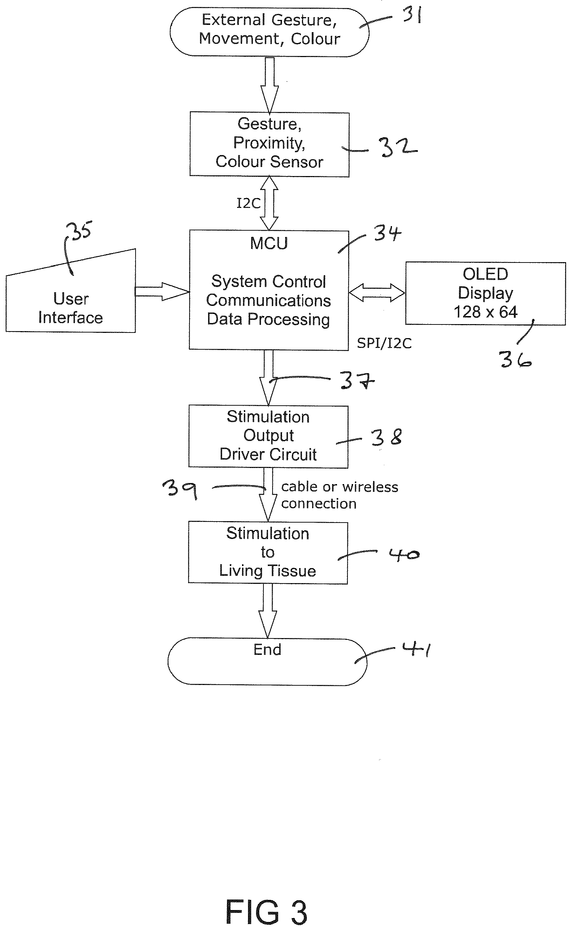

Figure 3 illustrates in a block diagram the steps used in processing in an apparatus in accordance with aspects of the present invention which uses electrical stimulation. Initially an external control feature 31 such as proximity, a gesture, movement or colour is presented and detected by an object sensor at a detection step 32. Signals 33 are exchanged between the object sensor and the micro controller unit (MCU) during a control feature processing step 34. The MCU will allow input 35 from a user interface through the switches mentioned above with respect to the housing 1 and depicted in Figure 1. The control housing in accordance with aspects of the present invention may include a display so that the status of the process can be displayed in a displaying step 36.

The MCU provides signals 37 to appropriate driver circuits at a stimulation parameter step 38 which provided driver signals 39, either wired through cable or wirelessly, to electrodes in contact with living tissue. The electrodes will present stimulation electrode signals in a stimulation step 40. Once the stimulation parameter or a sequence of parameters is completed the process ends at step 41 and the system resets ready for another control feature at step 31.

## Claims

1. A stimulator apparatus for providing stimulation to penile,scrotal, anal, vaginal or clitoral tissue of a human or animal body, said simulator apparatus comprising :-
(i) a control housing;
(ii) an electrical power source;
(iii) a contact connection;
(iv) an object sensor within the control housing adapted to detect at least one control feature, said control feature being one of
- proximity to the control housing,
- gesture,
- movement and
- colour
of an object and/or body part within a motion detection range of the object sensor within the control housing;
(v) control circuit means adapted to distinguish specific control features of the object or body part in sequence and to vary one or more stimulating parameters of an electrical output of the stimulator apparatus in response to the sequence of control features.

2. A stimulator apparatus according to Claim 1 wherein the control housing is in wired (cabled) or wireless connect with the contact connection.

3. A stimulator apparatus according to any preceding claim wherein the object is a household item or book and/or a body part is a finger or hand or foot of a user.

4. A stimulator apparatus according to any preceding claim wherein the apparatus includes display means to show status.

5. A stimulator apparatus according any preceding claim wherein the apparatus has more than one control housing with a respective object sensor.

6. A stimulator apparatus according to any preceding claim wherein the motion detection range is up to 10 centimetres

7. A stimulator apparatus according to any preceding claim wherein the contact connection comprises anode and cathode electrodes.

8. A stimulator apparatus according to any preceding claim wherein the contact connection comprises means to engage a skin surface to apply electrical stimulation.

## Patentansprüche

1. Stimulatorvorrichtung zur Bereitstellung von Stimulationen für Penis-, Skrotum-, Anal-, Vaginal- oder Klitorisgewebe eines menschlichen oder tierischen Körpers, wobei die Stimulatorvorrichtung umfasst:
(i) ein Steuergehäuse;
(i) eine elektrische Energiequelle;
(iii) einen Kontaktanschluss;
(iv) einen Gegenstandssensor innerhalb des Steuergehäuses, der dazu geeignet ist, zumindest ein Steuermerkmal zu erfassen, wobei das Steuermerkmal eines von
- Nähe zu dem Steuergehäuse,
- Geste,
- Bewegung und
- Farbe
eines Gegenstands oder Körperteils innerhalb eines Bewegungserfassungsbereichs des Gegenstandssensors innerhalb des Steuergehäuses ist;
(v) Steuerkreismittel, die dazu geeignet sind, spezifische Steuermerkmale des Gegenstands oder Körperteils in der Abfolge zu unterscheiden und einen oder mehrere Stimulationsparameter eines elektrischen Ausgangs der Stimulatorvorrichtung in Ansprechen auf die Abfolge von Steuermerkmalen zu variieren.

2. Stimulatorvorrichtung nach Anspruch 1, wobei das Steuergehäuse in drahtgebundener (kabelgebundener) oder drahtloser Verbindung mit dem Kontaktanschluss steht.

3. Stimulatorvorrichtung nach einem der vorhergehenden Ansprüche, wobei der Gegenstand ein Haushaltsgegenstand oder Buch ist und/oder ein Körperteil ein Finger oder eine Hand oder ein Fuß eines Benutzers ist.

4. Stimulatorvorrichtung nach einem der vorhergehenden Ansprüche, wobei die Vorrichtung Mittel zur Statusanzeige umfasst.

5. Stimulatorvorrichtung nach einem der vorhergehenden Ansprüche, wobei die Vorrichtung mehr als ein Steuergehäuse mit einem jeweiligen Gegenstandssensor aufweist.

6. Stimulatorvorrichtung nach einem der vorhergehenden Ansprüche, wobei der Bewegungserfassungsbereich bis zu 10 Zentimeter beträgt.

7. Stimulatorvorrichtung nach einem der vorhergehenden Ansprüche, wobei der Kontaktanschluss Anoden- und Kathoden-Elektroden umfasst.

8. Stimulatorvorrichtung nach einem der vorhergehenden Ansprüche, wobei der Kontaktanschluss Mittel umfasst, um mit einer Hautoberfläche zur Anwendung einer elektrischen Stimulation in Eingriff zu gelangen.

## Revendications

1. Appareil stimulateur pour fournir une stimulation au tissu pénien, scrotal, anal, vaginal ou clitoridien d'un corps humain ou animal, ledit appareil simulateur comprenant
(i) un boîtier de commande ;
(ii) une source d'alimentation électrique ;
(iii) une connexion de contact ;
(iv) un capteur d'objet dans le boîtier de commande adapté pour détecter au moins une caractéristique de commande, ladite caractéristique de commande étant l'un parmi
- la proximité du boîtier de commande,
- un geste,
- un mouvement et
- une couleur
d'un objet et/ou d'une partie du corps dans une plage de détection de mouvement du capteur d'objet dans le boîtier de commande ;
(v) des moyens de circuit de commande adaptés pour distinguer des caractéristiques de commande spécifiques de l'objet ou de la partie du corps en séquence et pour faire varier un ou plusieurs paramètres de stimulation d'une sortie électrique de l'appareil stimulateur en réponse à la séquence de caractéristiques de commande.

2. Appareil stimulateur selon la revendication 1 dans lequel le boîtier de commande est en connexion filaire (câblée) ou sans fil avec la connexion de contact.

3. Appareil stimulateur selon l'une quelconque des revendications précédentes dans lequel l'objet est un article ménager ou un livre et/ou une partie du corps est un doigt ou une main ou un pied d'un utilisateur.

4. Appareil stimulateur selon l'une quelconque des revendications précédentes dans lequel l'appareil comprend un moyen d'affichage pour afficher l'état.

5. Appareil stimulateur selon l'une quelconque des revendications précédentes dans lequel l'appareil a plus d'un boîtier de commande avec un capteur d'objet respectif.

6. Appareil stimulateur selon l'une quelconque des revendications précédentes dans lequel la plage de détection de mouvement va jusqu'à 10 centimètres.

7. Appareil stimulateur selon l'une quelconque des revendications précédentes dans lequel la connexion de contact comprend des électrodes d'anode et de cathode.

8. Appareil stimulateur selon l'une quelconque des revendications précédentes dans lequel la connexion de contact comprend un moyen pour s'engager avec une surface cutanée pour appliquer une stimulation électrique.
